# EUROPÄISCHE PATENTSCHRIFT

(11) **EP 2 529 679 B1**
(45) Veröffentlichungstag und Bekanntmachung des Hinweises auf die Patenterteilung: **14.05.2014**
(21) Anmeldenummer: 12170016.5
(22) Anmeldetag: 30.05.2012
(51) Int. Cl.: A61B 17/225

(54) **Schalldämpfungshülse zum Aufsetzen auf ein Druckwellengerät**
Acoustic dampening casing for application to a pressure wave device
Manchon d'amortissement du bruit à mettre en place sur un appareil à ondes de pression

(30) Priorität: 30.05.2011 DE 202011101571 U
(43) Veröffentlichungstag der Anmeldung: 05.12.2012
(73) Patentinhaber: STORZ MEDICAL AG, 8274 Tägerwilen (CH)
(72) Erfinder: MARLINGHAUS, Ernst H., 8598 Bottighofen (CH); NOVAK, Pavel, 8234 Stetten (CH); KATONA, Josef, 78315 Radolfzell (DE); SCHULZ, Manfred, 8274 Tägerwilen (CH); MÜLHAUSER, Peter, 8267 Berlingen (CH)
(74) Vertreter: Szynka, Dirk

(56) Entgegenhaltungen:
- WO-A1-99/08598
- DE-A1-102004 042 895
- DE-U1-202007 007 922
- US-A- 6 039 694
- US-A1- 2006 173 331
- "SIMRIT eCatalog Spezialdichtungsprodukte 2009", , 31. Dezember 2009 (2009-12-31), Seite 1, XP55036144, www.simrit.de Gefunden im Internet: URL:http://www.simrit.de/mimes/pd/Simrit_K ataloge/Medien2/DMS-SHOWCASES/MB_SHOW_SIMR IT/InDesign/highres-pdf/00000C83.pdf [gefunden am 2012-08-23]

## Beschreibung

Die vorliegende Erfindung betrifft eine Schalldämpfungshülse zum Aufsetzen auf ein Druckwellengerät zur Behandlung des menschlichen oder tierischen Körpers durch mechanische Druckwellen.

Geräte zur Behandlung mit mechanischen Druckwellen sind an sich bekannt, insbesondere aus dem Bereich der Lithotripsie. Dort werden mit fokussierten mechanischen Druckwellen Körperkonkremente, etwa Steine im Körpergewebe, zertrümmert. Neben der Erzeugung durch elektrische Entladungen im Wasser sind auch Geräte entwickelt worden, die mechanische Druckwellen durch das Aufeinanderprallen eines beschleunigten Schlagteils und eines Prallkörpers erzeugen. Solche Geräte werden sowohl in der Lithotripsie als auch bei anderen Behandlungen biologischer Körpersubstanzen eingesetzt, wobei insbesondere die Behandlung von Muskelerkrankungen und von Erkrankungen im Übergangsbereich zwischen Muskeln und Knochen sowie Anwendungen im dermatologischen Bereich zu nennen sind.

In der DE 10 2004 042 895 A1 ist ein Druckwellengerät mit einem pneumatisch beschleunigten Schlagteil offenbart, welches auf einen Prallkörper prallt und so die Druckwelle erzeugt. Der Prallkörper ist dabei mit einer auf das distale Ende des Druckwellengeräts gesetzten Hygienefolie abgedeckt, die am Gehäuse des Druckwellengeräts gehalten ist und nach der Behandlung ausgetauscht wird, um eine aufwendige Reinigung des Prallkörpers zu vermeiden.

Die WO 99/08598 A1 beschreibt einen Aufsatz für eine Ultraschallsonde, der einer Verunreinigung der Sonde vorbeugen soll und deshalb den aktiven Sondenkopf abdeckt.

Die US 6,039,694 A zeigt ein Hygieneset für Ultraschallgeräte, und zwar eine den aktiven Sondenkopf bedeckende Folie und eine diese am Gerät haltende Klammer. In der US 2006/0173331 A1 sind unterschiedliche Gehäuseformen einer Ultraschallsonde offenbart, wobei ein Griffbereich speziell auf eine zum Teil zur Faust geschlossene Hand abgestimmt ist.

Der vorliegenden Erfindung liegt das technische Problem zugrunde, die Gebrauchseigenschaften von Druckwellengeräten mit Schlagteil und Prallkörper zu verbessern.

Dieses technische Problem wird erfindungsgemäß durch die Verwendung einer rohrförmigen Schalldämpfungshülse mit offenen Enden gelöst, die auf das Gehäuse eines Druckwellengeräts gesetzt wird und dabei in Einkoppelrichtung eine Länge hat, die mindestens 20% der in derselben Richtung genommenen Länge eines sich innerhalb des Gehäuses erstreckenden Teils des Prallkörpers beträgt.

Die Erfindung richtet sich gleichermaßen auf ein Druckwellengerät mit beweglichem Schlagteil und Prallkörper, die in einem Gehäuse des Druckwellengeräts montiert sind, wobei an dem Gehäuse außenseitig eine erfindungsgemäße Schalldämpfungshülse vorgesehen ist.

Bevorzugte Ausgestaltungen der Erfindung sind in den abhängigen Ansprüchen angegeben, wobei in der gesamten Offenbarung nicht im Einzelnen zwischen der Beschreibung der Schalldämpfungshülse, des Druckwellengeräts und dem Verwendungsaspekt der Erfindung unterschieden wird; die Offenbarung ist implizit im Hinblick auf sämtliche Kategorien zu verstehen.

Die eingangs in Zusammenhang mit der Längenbestimmung genannte Einkoppelrichtung entspricht einer Hauptausbreitungsrichtung der Druckwellen, die sich nach einer Mittelung auch als Schwerpunktrichtung einer Vielzahl Ausbreitungsrichtungen ergeben kann, etwa im Falle sich radial ausbreitender Druckwellen. Wenn der Prallkörper in Abhängigkeit von seiner Lagerung im Gehäuse in Folge eines Aufpralls des Schlagteils ausgelenkt wird, fällt eine Richtungskomponente, typischerweise eine Hauptrichtungskomponente, dieser Auslenkung mit der Hauptausbreitungsrichtung zusammen. Der Übersichtlichkeit halber wird im Folgenden allein von der Einkoppelrichtung gesprochen.

Die angegebene Mindestlänge der Schalldämpfungshülse ist auf den Prallkörper bezogen, und zwar auf die in Einkoppelrichtung genommene Länge seines innerhalb des Gehäuses angeordneten Teils. Aus dem Gehäuse hervorstehende Teile des Prallkörpers bleiben bei der Längenbetrachtung somit außer Acht, der berücksichtigte Teil des Prallkörpers ist in zur Einkoppelrichtung senkrechten Schnittebenen von Gehäuse umgeben (nicht zwingend vollständig, jedoch vorzugsweise).

Die Erfinder haben festgestellt, dass es zu einer Geräuschentstehung kommt, selbst wenn eine einer Prallfläche für das Schlagteil entgegensetzte Seite des Prallkörpers an dem zu behandelnden Körperteil anliegt und die Schallausbreitung dadurch eigentlich gedämpft sein sollte. Dies kann zu einer erheblichen Geräuschbelästigung führen, sowohl für den Behandelnden als auch für den Patienten.

Durch die erfindungsgemäße Schalldämpfungshülse wird die Ausbreitung des sich in Folge des Aufeinanderprallens von Schlagteil und Prallkörper auch quer zur Einkoppelrichtung ausbreitenden Schalls eingedämmt bzw. gedämpft. Es kommt hierbei sowohl eine vielfach als Schalldämmung bezeichnete Behinderung der Schallausbreitung durch Reflexion an Grenzflächen infrage als auch eine oftmals als Schalldämpfung bezeichnete Behinderung der Schallausbreitung durch Absorption von Schall (gleichwohl wird im Kontext dieser Offenbarung der Einfachheit halber allein von Schalldämpfung gesprochen).

Sofern etwa bei der Beschreibung der Schalldämpfungshülse auf das Druckwellengerät oder einen zu behandelnden Körper Bezug genommen wird, impliziert dies nicht, dass zur Erfüllung des Erfindungsgegenstands die Schalldämpfungshülse auch tatsächlich auf ein Druckwellengerät gesetzt bzw. dieses gegenüber einem Körper entsprechend positioniert werden muss. Es wird vielmehr allein die Geometrie beschrieben, als ob die Schalldämpfungshülse aufgesetzt wäre bzw. ein Körper behandelt würde. "Körper" meint ohne gegenteilige Angabe den Körper des Patienten, also des Behandelten.

Durch die außenseitig am Gehäuse anliegende Schalldämpfungshülse kann die seitliche Schallausbreitung vorteilhafterweise reduziert werden, wobei erfahrungsgemäß eine gewisse Mindestlänge der Schalldämpfungshülse einzuhalten ist.

Vorzugsweise ist die Länge der Schalldämpfungshülse in Einkoppelrichtung größer als in dieser Reihenfolge zunehmend bevorzugt 25%, 50%, 75% oder sogar 100% der in derselben Richtung genommenen Länge des sich innerhalb des Gehäuses erstreckenden Teils des Prallkörpers, besonders bevorzugt ist die Länge der Schalldämpfungshülse in Einkoppelrichtung also größer als die in derselben Richtung genommene Länge des sich innerhalb des Gehäuses erstreckenden Teils des Prallkörpers. Im Folgenden wird der Einfachheit halber von der Länge des Prallkörpers gesprochen, wenngleich außer im Falle einer ausdrücklich anderen Angabe die Länge des sich innerhalb des Gehäuses erstreckenden Teils davon gemeint ist. Der Prallkörper kann dann beispielsweise in Bezug auf Richtungen senkrecht zur Einkoppelrichtung vollständig von der Schalldämpfungshülse umgeben sein, sodass der sich vom Prallkörper seitwärts ausbreitende Schall über die gesamte Länge des Prallkörpers gedämpft wird.

In bevorzugter Ausgestaltung ist die Schalldämpfungshülse für eine solche Anlage an dem Gehäuse ausgelegt, dass sie einen für das Aufprallen des Schlagteils vorgesehenen Bereich des Prallkörpers senkrecht zur Einkoppelrichtung nach außen abdeckt. Eine zur Einkoppelrichtung senkrechte, zu der für das Aufprallen des Schlagteils vorgesehenen Prallfläche des Prallkörpers tangentiale "Ebene des Aufpralls" schneidet also die Schalldämpfungshülse. Besonders bevorzugt schneidet die Ebene des Aufpralls die Schalldämpfungshülse in Bezug auf die Einkoppelrichtung in einem mittigen Bereich, sodass die Ebene des Aufpralls die Schalldämpfungshülse in zwei Teile teilt und sich jede der Hälften auf mindestens 25%, besonders bevorzugt mindestens 35%, der gesamten Schalldämpfungshülsenlänge bemisst.

Die Schalldämpfungshülse ist als rohrförmiger Körper ausgebildet, der beispielsweise im Falle eines zylindersymmetrischen Gehäuses eine kreisringförmige Querschnittsform haben kann. Die Enden des rohrförmigen Körpers sind offen ausgebildet.

Eine zu dem Körper hin offene Schalldämpfungshülse kann in Abhängigkeit von ihrer Position auf dem Gehäuse auch eine direkte Anlage des Prallkörpers an den Körper und eine dementsprechend gute Einkopplung der Druckwellen ermöglichen. Die Schalldämpfungshülse wird vorzugsweise so auf dem Gehäuse angeordnet, dass sie auch einen aus dem Gehäuse hervorstehenden Teil des Prallkörpers senkrecht zur Einkoppelrichtung abdeckt, was die von diesem ansonsten (von dem Gehäuse) nicht abgeschirmten Bereich ausgehende Schallausbreitung reduzieren hilft.

Die Einkopplung kann auch durch das Aufbringen eines Koppelgels verbessert werden, welches dann während der Behandlung als dünner Film zwischen der Einkoppelfläche des Prallkörpers und dem Körper vorliegt. In diesem Zusammenhang kann eine weitere bevorzugte Ausführungsform Vorteile bieten, wenn nämlich die Schalldämpfungshülse im Bereich des dem Körper zugewandten offenen Endes solchermaßen von dem Gehäuse beabstandet ist, dass ein Schalldämpfungshülseninnenwandbereich zusammen mit dem Gehäuse ein Volumen begrenzt. In dieses kann dann nämlich ein Koppelgel eingefüllt werden, vorzugsweise vor einer Behandlung. Weiter bevorzugt ist das von Schalldämpfungshülseninnenwandbereich und

Gehäuse begrenzte Volumen zu dem offenen Ende der Schalldämpfungshülse hin, also zu dem Körper hin, offen.

Die Befüllung mit Koppelgel kann durch das zu dem Körper hin offene Ende der Schalldämpfungshülse erfolgen; vorzugsweise ist dazu eine eigene Öffnung vorgesehen, die beispielsweise schräg zur Einkoppelrichtung orientiert von einem Hülsenaußenwandbereich her einen Zugang zu dem Koppelgelreservoir ermöglicht. "Schräg" meint hierbei einen mit der Hauptausbreitungsrichtung eingeschlossenen Winkel (den kleineren von zwei zusammen 180° ergebenden Winkeln) von in dieser Reihenfolge zunehmend bevorzugt mindestens 30°, 45°, 60°, 75°, 85°.

Über die zusätzliche Öffnung kann dann sowohl ein zu dem Körper hin offenes Koppelgelreservoir als auch ein zu dem Körper hin geschlossenes befüllt werden. Die Koppelgelabgabe kann bei Letzterem etwa erfolgen, indem der Behandelnde auf einen dem Koppelgel entgegengesetzten Hülsenaußenwandbereich drückt und so durch den Druck des Koppelgels ein temporäres Öffnen des ansonsten geschlossenen Volumens bewirkt, etwa indem das Koppelgel zwischen einem ansonsten an dem Gehäuse anliegenden Kragen einer Schalldämpfungshülse aus Elastomermaterial und dem Gehäuse hindurchgedrückt wird.

Das Befüllen eines zu dem Körper hin offenen Volumens über eine zusätzliche Öffnung kann den Vorteil bieten, dass dabei Luft durch die dem Körper zugewandte Öffnung entweichen, das Reservoir also im Wesentlichen luftblasenfrei befüllt werden kann. In diesem Zusammenhang könnte eine zusätzliche Öffnung auch der Entlüftung dienen.

Die Erfinder haben festgestellt, dass das Koppelgel während einer Behandlung seitlich weggeschoben und somit üblicherweise innerhalb kurzer Zeit über die Haut verteilt wird. Dies hat zur Folge, dass ein behandelnder Arzt oder Therapeut das Gel zwischenzeitlich immer wieder zurückschieben oder neues Gel auftragen muss; in beiden Fällen ist die Behandlung beeinträchtigt bzw. muss diese unterbrochen werden.

Indem erfindungsgemäß eine Schalldämpfungshülse mit einem zu dem Körper hin offenen Reservoir vorgesehen wird, kann, wenn während der Behandlung das körperzugewandte offene Ende der Schalldämpfungshülse an dem Körper anliegt, der von dem Gehäuse beabstandete Innenwandbereich ein seitliches Wegschieben des Koppelgels zumindest teilweise unterbinden. Mit anderen Worten ist das ansonsten offene Volumen dann von der Haut des Patienten begrenzt, wird das Koppelgel während der Behandlung also in einem (innerhalb gewisser Grenzen) geschlossenen Volumen beisammengehalten. Es kann also beispielsweise weniger Koppelgel vorgesehen werden, was etwa in Kostenhinsicht vorteilhaft ist, bzw. kann die Aufmerksamkeit des Behandelnden auf die eigentliche Behandlung gerichtet bleiben.

Eine weitere Ausführungsform betrifft einen nach innen gerichteten umlaufenden Kragen, durch den die dem Körper zugewandte Öffnung der Schalldämpfungshülse verjüngt ist. Ein solcher Kragen kann beispielsweise beim Aufsetzen der Schalldämpfungshülse als Anschlag dienen und so eine Relativposition von Schalldämpfungshülse und Druckwellengerät vorgeben. Besonders bevorzugt wird der Kragen jedoch in Verbindung mit dem von dem Gehäuse beabstandeten Innenwandbereich, also dem Koppelgelreservoir, vorgesehen und kann so etwa das Koppelgel an dem offenen Ende in Richtung der Einkoppelfläche des Prallkörpers lenken. Der Kragen kann auch in der zuvor beschriebenen Weise an dem Gehäuse anliegen und das Koppelgelreservoir so zu dem Körper hin verschließen (und nur unter Druck öffnen und Koppelgel freigeben); vorzugsweise ist das Koppelgelreservoir jedoch auch im Falle eines vorgesehenen Kragens zu dem Körper hin offen, auch wenn kein Koppelgel gegen den Kragen drückt.

Wird beispielsweise eine Schalldämpfungshülse aus einem Elastomermaterial vorgesehen, kann das Volumen des Koppelgelreservoirs beim Andrücken der Schalldämpfungshülse an den zu behandelnden Körper verändert und so auch ein Herausdrücken des Koppelgels bewirkt werden. Dies kann etwa auch durch eine Relativverschiebung zwischen Schalldämpfungshülse (also des vom Gehäuse beabstandeten Bereichs davon) und Gehäuse senkrecht zur Einkoppelrichtung erfolgen.

Der Durchmesser der durch den Kragen verjüngten Öffnung ist vorzugsweise um mindestens 10%, in dieser Reihenfolge zunehmend bevorzugt um mindestens 15 %, 20 %, 25 %, 30%, kleiner als der Innendurchmesser des für die Anlage am Gehäuse vorgesehenen Bereichs der Schalldämpfungshülse (also auch gleichermaßen kleiner als der Außendurchmesser des Gehäuses). Wenngleich für Prallkörper, Gehäuse und Schalldämpfungshülse eine zylindrische Geometrie bevorzugt ist, sollen auch Ausführungsformen mit anderen Querschnittsformen umfasst sein und ist in einem solchen Fall die Durchmesserangabe auf den minimalen Durchmesser bezogen, also beispielsweise im Falle eines quadratischen Querschnitts auf die Kantenlänge.

Im Hinblick auf gute Schalldämpfungseigenschaften, insbesondere hinsichtlich einer Absorption von Schall, ist für die Schalldämpfungshülse ein Material mit einem Elastizitätsmodul von weniger als 10 GPa, in dieser Reihenfolge zunehmend bevorzugt von weniger als 5, 3, 2, 1 GPa, bevorzugt. Es kann beispielsweise eine Schalldämpfungshülse aus einem Elastomermaterial, etwa einem insbesondere auch synthetischen Kautschukmaterial, vorgesehen werden.

Im Hinblick auf die Schallabsorption ist auch eine gewisse Mindestdicke, gemessen in einer Richtung senkrecht zur Einkoppelrichtung, bevorzugt; die Schalldämpfungshülse hat in dieser Reihenfolge zunehmend bevorzugt eine Dicke von mindestens 1, 2, 3, 4 mm.

Die Erfinder haben festgestellt, dass die Schalldämpfungseigenschaften ferner durch in der bzw. von der Schalldämpfungshülse eingeschlossene Luftvolumina verbessert werden können, weil ein Luftvolumen als Unstetigkeitsstelle die Schalltransmission verringern helfen kann.

Hier hat sich eine Beabstandung der Schalldämpfungshülse von dem Gehäuse als vorteilhaft erwiesen, und zwar durch einen an der Schalldämpfungshülse innenseitig vorgesehenen Abstandshalter. So kann einerseits in einem Pfad der Schallausbreitung ein Luftvolumen zur Verfügung gestellt werden; andererseits kann ein solcher innenseitig vorgesehener Abstandshalter auch einfach hergestellt werden, etwa in einem formenden Verfahren in demselben Fertigungsschritt wie die Schalldämpfungshülse.

In weiterer Ausgestaltung ist der Abstandshalter als innenseitiger, vorzugsweise umlaufender Steg ausgebildet, sodass beispielsweise in Bezug auf die Einkoppelrichtung beidseits des Stegs ringförmige Luftvolumina zwischen Schalldämpfungshülse und Gehäuse eingeschlossen sein können. Die Angabe "ringförmig" bezieht sich hierbei auf eine zur Einkoppelrichtung senkrechte Schnittebene, wobei eine Kreisringform bevorzugt ist. Es können sich dann also beispielsweise mehrere ringförmige bzw. kreisringförmige Luftvolumina jeweils über eine bestimmte Länge in Einkoppelrichtung erstrecken und in Einkoppelrichtung direkt aufeinanderfolgende Luftvolumina jeweils durch einen umlaufenden Steg voneinander getrennt sein.

Mit einer Mehrzahl solcher durch Stege voneinander getrennten Luftvolumina kann nahezu über die gesamte Länge der Schalldämpfungshülse ein Luftvolumen in einem Pfad der Schallausbreitung vorgesehen werden und liegt die Schalldämpfungshülse gleichzeitig mit der Mehrzahl Stege an dem Gehäuse an, ist also hinreichend gegen ein Verrutschen gesichert.

In bevorzugter Ausgestaltung ist eine Schalldämpfungshülse mit einem Elastomerkörper vorgesehen, der zwischen Schalldämpfungshülse und Gehäuse angeordnet wird. Generell kann ein solcher Elastomerkörper beispielsweise die Schalldämpfungshülse von dem Gehäuse beabstanden und etwa in Verbindung mit einem weiteren Elastomerkörper ein in den vorstehenden Absätzen beschriebenes Luftvolumen begrenzen; bevorzugt ist gleichwohl ein einstückig mit der Schalldämpfungshülse ausgebildeter Abstandshalter.

Schalldämpfungshülse und Elastomerkörper sind hingegen mehrteilig, also nicht als einstückiger Körper ausgebildet. Vorzugsweise ist der Elastomerkörper ein umlaufender Ring, etwa ein Dichtring mit beispielsweise kreisringförmigem Querschnittsprofil (O-Ring). Für den Elastomerkörper kann zum Beispiel auch ein gegenüber jenem der Schalldämpfungshülse weicheres Material vorgesehen werden, dieses kann beispielsweise einen um jeweils mindestens 10 %, 25 % oder auch 50 % geringeren Elastizitätsmodul als das für die Schalldämpfungshülse vorgesehene Material haben. Der Elastomerkörper kann Gehäuse und Schalldämpfungshülse so beispielsweise auch mechanisch entkoppeln, was hinsichtlich der Schalldämpfung Vorteile bieten kann.

Vorzugsweise wird der Elastomerkörper in einer innenseitig der Schalldämpfungshülse, dem Gehäuse zugewandten Ausnehmung angeordnet, die den Elastomerkörper beispielsweise auch beim Aufschieben der Schalldämpfungshülse in Position halten kann; andererseits kann beispielsweise ein O-Ring das Aufschieben auch erleichtern. Über den Elastomerkörper kann die Schalldämpfungshülse beispielsweise auch zentriert auf dem Gehäuse gelagert sein.

Wenngleich im Allgemeinen auch der Elastomerkörper selbst die Schalldämpfungshülse von dem Gehäuse beabstanden kann, ist ein einstückig mit der Schalldämpfungshülse ausgebildeter Steg bevorzugt, in dem besonders bevorzugt eine Ausnehmung vorgesehen ist; in dieser ist der dann an dem Gehäuse anliegende Elastomerkörper angeordnet (die Schalldämpfungshülse wird dann gegebenenfalls durch Steg und Elastomerkörper von dem Gehäuse beabstandet werden, wobei der Anteil des Elastomerkörpers an der überbrückten Distanz üblicherweise geringer sein wird). Der Elastomerkörper kann also vorteilhafterweise ein vorstehend beschriebenes, vorzugsweise ringförmiges und besonders bevorzugt auch von einem Steg begrenztes Luftvolumen abdichten.

Die Geräuschentwicklung kann auf das Aufeinanderprallen von beweglichem Schlagteil und üblicherweise elastisch gelagertem Prallkörper zurückgeführt werden. Dabei kommt es während einer Behandlung typischerweise nicht nur zu einem solchen Aufprall, sondern prallen Schlagteil und Prallkörper wiederholt und vielfach aufeinander. Hierzu kann das Schlagteil beispielsweise auf einer Kreisbahn bewegt werden und mit jeder Umdrehung den Prallkörper streifen. Das Schlagteil kann beispielsweise auch auf einer linearen Bahn beschleunigt werden, etwa elektromagnetisch oder mit einem Federmechanismus.

Generell ist ein pneumatischer Antrieb, mit dem das Schlagteil bewegbar ist, bevorzugt; es könnte also beispielsweise eine Achse, mit der das Schlagteil verbunden ist, mit Druckluft in Rotation versetzt und das Schlagteil so auf einer Kreisbahn bewegt werden.

Besonders bevorzugt ist das Schlagteil jedoch in einem sich in Einkoppelrichtung erstreckenden Führungsrohr geführt, "passt" das Schlagteil also in Richtungen senkrecht zur Einkoppelrichtung mit beispielsweise geringem Spiel in das Führungsrohr und kann durch einen pneumatischen Druckpuls des pneumatischen Antriebs in Einkoppelrichtung bewegt werden.

Die Erfindung richtet sich auch auf die Verwendung einer vorstehend beschriebenen Schalldämpfungshülse zum Aufsetzen auf ein solches Druckwellengerät. Dabei kann in bevorzugter Ausgestaltung die Schalldämpfungshülse auch als Einwegbauteil vorgesehen sein, das vor einer Behandlung eines weiteren Patienten gegen eine unbenutzte Schalldämpfungshülse ausgetauscht wird.
- Fig. 1: zeigt ein an sich vorbekanntes pneumatisch betriebenes Druckwellengerät mit Prallkörper und Schlagteil in einem Längsschnitt.
- Fig. 2: zeigt das dem Körper zugewandte Ende eines Druckwellengeräts gemäß Figur 1 mit dem Prallkörper in vergrößerter Darstellung, wobei eine erfindungsgemäße Schalldämpfungshülse aufgesetzt ist.
- Fig. 3: zeigt verschiedene Ansichten einer erfindungsgemäßen Schalldämpfungshülse.
- Fig. 4: illustriert analog zu Figur 2 eine auf das dem Körper zugewandte Ende eines Druckwellengeräts aufgesetzte erfindungsgemäße Schalldämpfungshülse.

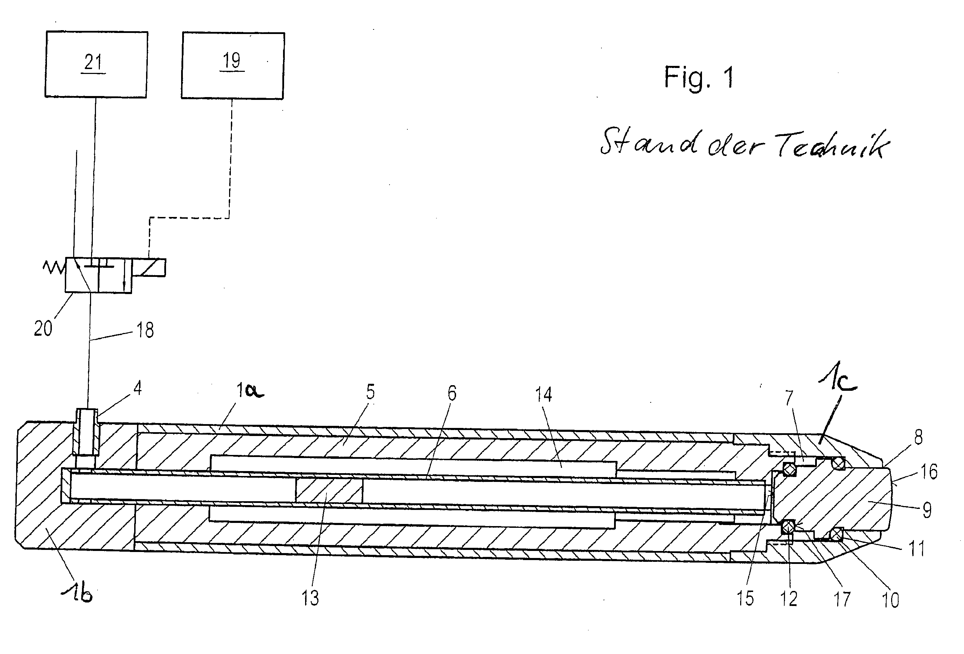

In Figur 1 ist in einem Schnitt entlang einer Längsachse ein Gerät zur Einkopplung von fokussierten mechanischen Druckwellen in beispielsweise den menschlichen Körper dargestellt. Ein Rohrstück 1a bildet gemeinsam mit einer in der Anwendung körperabgewandten Zuluftkappe 1 b und einer in der Anwendung körperzugewandten Applikatorkappe 1c ein Gehäuse 1.

Die Zuluftkappe 1 b enthält einen Druckluftanschluss 4 für eine pneumatische Versorgung. In an sich bekannter Weise ist an diesen Druckluftanschluss 4 über eine pneumatische Versorgungsleitung 18 ein von einer Ansteuereinheit 19 gesteuertes Ventil 20, insbesondere Magnetventil, angeschlossen, das in einem gleich bleibenden iterativen Takt zwischen etwa 1 Hz und 50 Hz Druckluftpulse über den Druckluftanschluss einkoppelt.

Das Gerät ist als mit der Hand einer Bedienungsperson zu haltendes Gerät ausgebildet, das über die erwähnte Pneumatikleitung 18 an eine Basisstation mit der Ansteuereinheit 19 und dem Kompressor 21 angeschlossen ist und auf den Patienten manuell aufgesetzt werden kann. Es dient zur Behandlung von Weichgewebe, insbesondere Muskeln.

In dem Gehäuse 1 ist über einen Einsatz 5 ein Führungsrohr 6 gehalten, dessen bei der Anwendung körperfernes Ende in der Zuluftkappe 1 b endet und dort mit dem Druckluftanschluss 4 kommuniziert. Das in der Anwendung körperseitige Ende des Führungsrohres 6 endet in einem Teil des Einsatzes 5, der in die Applikatorkappe 1c hineinragt, und zwar kurz vor dem dortigen Ende des Einsatzes 5 und einem Innenraum 7 in der Applikatorkappe 1 c.

In dem Innenraum 7, der mit zwei radialen Schultern in eine in der Anwendung körperseitige Applikatoröffnung 8 übergeht, ist ein Prallkörper 9 aufgenommen. Dieser stützt sich über einen O-Ring 10 aus einem Elastomer an einer der radialen Schultern ab und weist hierzu einen Flansch 11 auf. Ein zur körperfernen Seite gerichtetes Ende 15 des Prallkörpers 9 stützt sich über einen weiteren O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einem Flansch 17 bzw. einer Schulter des Prallkörpers 9. Die Applikatoröffnung 8 dient dabei zu einer in der Längsrichtung verschiebbaren Führung des Prallkörpers 9 und fixiert diesen quer zur Längsrichtung. Die Axialverschiebbarkeit ist durch die Nachgiebigkeit der Elastomerringe 10 und 12 begrenzt und liegt bei einem in Luft betriebenen Gerät relativ zum Restgerät deutlich über 0,6 mm.

Der Prallkörper 9, der hier gleichzeitig den auf die Haut aufzusetzenden Applikator bildet, ist durch Abschrauben der Applikatorkappe 1 c austauschbar.

In dem angrenzenden Bereich des Führungsrohres 6 ist ein in Figur 1 mit dem Prallkörper 9 in Kontakt stehendes Schlagteil 13 eingesetzt. Dieses passt (in Bezug auf das Führungsrohr und die im Wesentlichen zylindrische Geometrie des Schlagteils 13) radial mit geringem Spiel. Das Schlagteil 13 kann durch Druckunterschiede der Luftsäule in dem Führungsrohr 6 vor und hinter ihm (d. h. in Figur 1 rechts und links des Schlagteils 13) in dem Führungsrohr hin- und herbewegt werden und insbesondere auf den Prallkörper 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Prallkörper 9 zugewandten Frontfläche (in Figur 1 der Übersichtlichkeit halber nicht bezeichnet) auf den Prallkörper 9 auf, und zwar auf eine körperabgewandte Prallfläche 15 davon.

Die Rückbewegung des Schlagteils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Schlagteils 13 in Richtung zu dem Prallkörper 9 verdrängt und damit dort komprimiert. Wenn das Magnetventil 20 in der Pneumatikzuleitung 18 des Druckluftanschlusses 4 den Druck wegschaltet, wird das Schlagteil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Schlagteil 13 erfolgen. Das in der Anwendung körperferne Ende des Führungsrohres 6 endet in einem Magnethalter 17 für das Schlagteil 13.

Der Prallkörper 9 hat eine rotationssymmetrische Zylinderform und ist in axialer Richtung durch die Prallfläche 15 und die etwas konvexe Einkoppelfläche 16 begrenzt. Der Außenmantel weist die bereits beschriebenen flanschartigen Strukturen 11 und 17 auf, die Anlageschultern für die O-Ringe 10 und 12 bilden. Im Übrigen ist ein austrittsseitiger Teil der Zylindergeometrie mit konstantem Radius gestaltet und damit in der Öffnung 8 axial verschiebbar.

Prallkörper wie der hier gezeichnete Prallkörper 9 können sich hinsichtlich Form, Material und Lagerung erheblich unterschieden. So gibt es unterschiedliche fokussierende und nicht fokussierende Formen und verschiedene gewölbte Austrittsflächen im Stand der Technik. Ferner kommen verschiedene Materialien in Betracht, etwa Edelstahl, Titan, und verschiedene Keramiken wie Siliziumnitrid sowie Kunststoffe. Schließlich können verschiedene Prallkörper unterschiedlich hart gelagert sein und dabei unterschiedliche Hübe ausführen, also eine unterschiedlich lange makroskopische Bewegung bei der Einkopplung der Druckwelle durchführen.

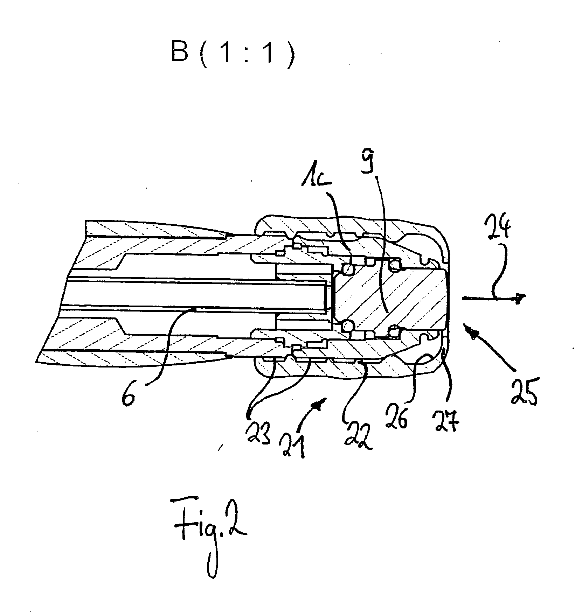

Figur 2 zeigt in einer Schnittdarstellung das körpernahe Ende des eben erläuterten Druckwellengeräts mit der Applikatorkappe 1c, dem innerhalb elastisch gelagerten Prallkörper 9 und dem Führungsrohr 6 in vergrößerter Darstellung. An dem mit durch Applikatorkappe 1c und Rohrstück 1a gebildeten Gehäuse 1 ist außenseitig eine erfindungsgemäße Schalldämpfungshülse 21 vorgesehen.

Die Schalldämpfungshülse 21 liegt mit innenseitig umlaufenden Stegen 22 an dem Gehäuse 1 an. Die Stege 22 beabstanden die Schalldämpfungshülse 21 von dem Gehäuse 1, sodass zwischen Gehäuse 1 und Schalldämpfungshülse 21 ringförmige, jeweils durch einen Steg 22 voneinander getrennte Luftvolumina 23 eingeschlossen sind. Da Schallwellen an Grenzflächen zwischen verschiedenen Medien zumindest teilweise reflektiert werden, kann die Schallausbreitung durch die ringförmigen Luftvolumina 23 in radialer Richtung gedämpft werden.

Für die Schalldämpfungshülse 21 ist hinsichtlich einer guten Schalldämpfung, insbesondere im Hinblick auf eine gute Schallabsorption, ein Elastomermaterial vorgesehen, nämlich Silikonkautschuk. Ferner ist in diesem Zusammenhang eine gewisse Mindestdicke vorteilhaft, weswegen die senkrecht zur Einkoppelrichtung 24 genommene Dicke der Schalldämpfungshülse mindestens 4 mm beträgt (und im Bereich eines Stegs 22 entsprechend größer ist). Diese Mindestdickenangabe bezieht sich dabei vorrangig auf einen in Bezug auf die Einkoppelrichtung 24 mittigen Bereich, also jedenfalls auf einen zwischen 30 % und 70% der in Einkoppelrichtung 24 genommenen Länge der Schalldämpfungshülse 21 liegenden Bereich.

Im Bereich einer endseitigen, dem Körper zugewandten Öffnung 25 der Schalldämpfungshülse 21 ist ein Schalldämpfungshülseninnenwandbereich 26 von der Applikatorkappe 1c, also von dem Gehäuse 1, beabstandet, sodass die Schalldämpfungshülse 21 gemeinsam mit dem Gehäuse 1 ein zu dem offenen Ende 25 der Schalldämpfungshülse 21 hin offenes Volumen begrenzt. In dieses kann ein Therapeut vor einer Behandlung ein Koppelgel einfüllen. Während der Behandlung begrenzt dann die Schalldämpfungshülse 21 einerseits eine seitliche Verteilung des Koppelgels, hält es also auf die zu behandelnde Stelle konzentriert. Andererseits kann aus dem dann als Koppelgelreservoir dienenden Volumen im Zuge der Behandlung fortwährend auch etwas Koppelgel abgegeben werden, etwa wenn das körperzugewandte Ende der Schalldämpfungshülse 21 und damit das innerhalb begrenzte Volumen beim Andrücken an den Körper verformt werden, das Koppelgel also aus dem Koppelgelreservoir herausgedrückt wird.

Um das Koppelgel in Richtung der Einkoppelfläche 16 des Prallkörpers 9 zu lenken, ist die körperzugewandte Öffnung 25 durch einen nach innen gerichteten, umlaufenden Kragen 27 verjüngt. Durch den Kragen 27 wird die körperzugewandte Öffnung des Koppelgelreservoirs generell verringert, sodass das Koppelgel in dem Reservoir auch für eine längere Zeitdauer bevorratet bleibt, also eine Abgabe über einen längeren Behandlungszeitraum möglich ist.

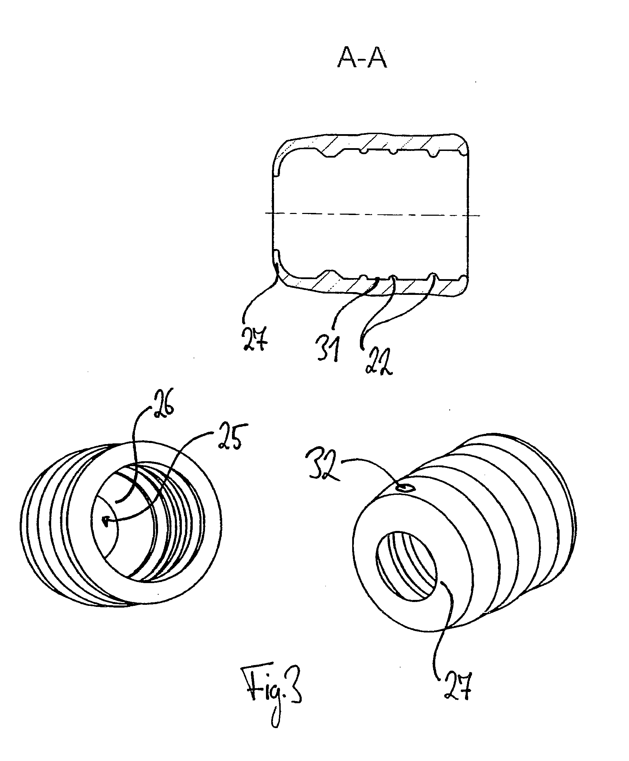

Fig. 3 zeigt oben eine erfindungsgemäße Schalldämpfungshülse 21 in einem Schnitt entlang einer Längsachse, links unten in einer Schrägansicht auf das körperabgewandte Ende blickend und rechts unten in einer Schrägansicht auf das körperzugewandte Ende blickend. In der zuletzt genannten Schrägansicht ist eine Öffnung 32 zu erkennen, die in das Koppelreservoir mündet und zum Einfüllen des Koppelgels dient.

Die körperzugewandte Öffnung 25 ist durch den Kragen 27 gegenüber einem Au-ßendurchmesser des Gehäuses, der 30 mm beträgt, um mehr als 30% verjüngt und bemisst sich somit auf etwa 19 mm.

Die innenseitig umlaufenden Stege 22 erheben sich von einer Schalldämpfungshülseninnenwand um 1 mm nach innen. Die dann von dem Gehäuse 1 und der Schalldämpfungshülse 21 eingeschlossenen, ringförmigen Luftvolumina 23 haben somit senkrecht zur Einkoppelrichtung 24 eine Dicke von 1 mm.

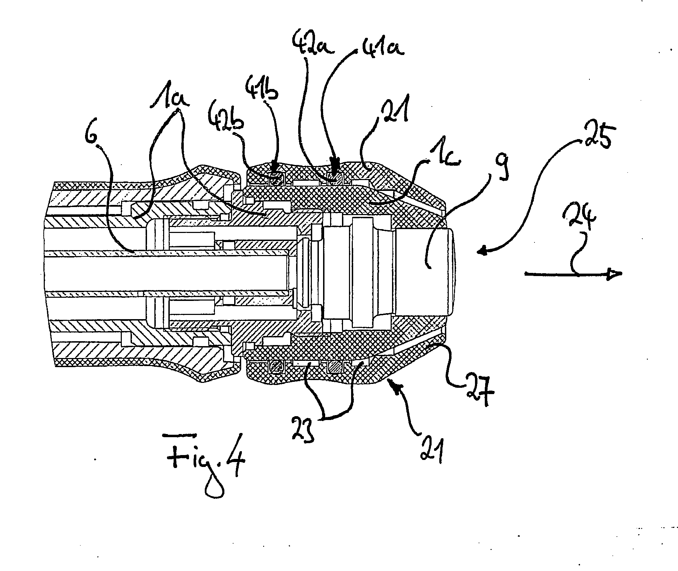

Figur 4 zeigt in einer Schnittdarstellung das körpernahe Ende eines Druckwellengeräts mit Applikatorkappe 1c, innerhalb elastisch gelagertem Prallkörper 9 (die elastische Lagerung ist der Übersichtlichkeit halber nicht dargestellt) und Führungsrohr 6.

Applikatorkappe 1c und Rohrstück 1a bilden das Gehäuse 1, an dem außenseitig eine erfindungsgemäße Schalldämpfungshülse 21 vorgesehen ist.

Dabei sind zwischen Applikatorkappe 1c und Schalldämpfungshülse 21 wiederum ringförmige Luftvolumina 23 angeordnet, welche die Schallausbreitung dämpfen können. Im Bereich der dem Körper zugewandten Öffnung 25 der Schalldämpfungshülse 21 ist diese ferner analog jener aus Figur 2 von dem Gehäuse 1 beabstandet, begrenzt die Schalldämpfungshülse 21 also gemeinsam mit dem Gehäuse 1 ein zu dem offenen Ende 25 der Schalldämpfungshülse 21 hin offenes Volumen, in welches Koppelgel eingefüllt werden kann.

Um das Koppelgel auf den Kontaktbereich zwischen Prallkörper 9 und zu behandelndem Körper zu konzentrieren, ist die Öffnung 25 durch den nach innen gerichteten Kragen 27 verjüngt. Im Gegensatz zu der anhand von Figur 2 erläuterten Schalldämpfungshülse 21 ist der Kragen 27 vorliegend nicht als sich zunächst von der Oberfläche der Applikatorkappe 1c weg erhebender Bogen (der ein entsprechend größeres Volumen für das Koppelgel begrenzt) ausgebildet, sondern erstreckt sich der Kragen im Wesentlichen parallel zur Oberfläche der Applikatorkappe 1 c.

In Figur 4 erstreckt sich der Kragen in Einkoppelrichtung 24 nicht ganz bis zu dem körperzugewandten Ende des Prallkörpers 9; dies wäre jedoch auch möglich, sodass der Kragen analog zu der in Figur 2 gezeigten Schalldämpfungshülse 21 bündig mit dem Prallkörper abschließen oder diesen in Einkoppelrichtung sogar auch überragen könnte. Ferner könnte sich die Schalldämpfungshülse (was in der Figur gleichermaßen nicht gezeigt ist) auch in der entgegengesetzten Richtung weiter erstrecken und insbesondere den Kontaktbereich von Applikatorkappe 1c und Rohrelement 1a vollständig überdecken, also in der Figur zumindest noch die Nut ausfüllen oder sich auch noch weiter über das Gehäuse 1 erstrecken (vom Körper weg).

Der die ringförmigen Luftvolumina 23 voneinander trennende Steg 22 weist vorliegend eine innenseitig umlaufende Ausnehmung 41 a auf, in welcher ein O-Ring 42a angeordnet ist. An dem dem zu behandelnden Körper abgewandten Ende der Schalldämpfungshülse 21 ist ferner eine zweite innenseitig umlaufende Ausnehmung 41 b mit darin angeordnetem zweiten O-Ring 42b vorgesehen. Die O-Ringe 42a, b können einerseits schon eine Abdichtung des dazwischen angeordneten Luftvolumens 23 sicherstellen; andererseits können die O-Ringe 42a, b beispielsweise auch das Aufschieben der Schalldämpfungshülse 21 auf die Applikatorkappe 1c erleichtern und sie auf dieser zentrieren.

## Patentansprüche

1. Gerät zur Behandlung des menschlichen oder tierischen Körpers durch mechanische Druckwellen mit
einem beweglichen Schlagteil (13),
einem Prallkörper (9), wobei die Druckwellen durch Beschleunigen und Aufprallen des Schlagteils (13) auf den Prallkörper (9) erzeugbar und in den Körper einkoppelbar sind,
und einem Gehäuse (1), in dem das Schlagteil (13) und der Prallkörper (9) montiert sind, und
einer Schalldämpfungshülse (21),
wobei die Schalldämpfungshülse (21) für eine Anlage außenseitig an dem Gehäuse (1) ausgelegt ist und dabei in Einkoppelrichtung (24) eine Länge hat, die mindestens 20% der in derselben Richtung genommenen Länge eines sich innerhalb des Gehäuses (1) erstreckenden Teils des Prallkörpers (9) beträgt,
**dadurch gekennzeichnet, dass**
die Schalldämpfungshülse (21) als rohrförmiger Körper mit offenen Enden ausgebildet ist.

2. Gerät nach Anspruch 1, bei welchem die Schalldämpfungshülse (21) für eine solche Anlage an dem Gehäuse (1) ausgelegt ist, dass sie einen für das Aufprallen des Schlagteils (13) vorgesehenen Bereich des Prallkörpers (9) senkrecht zur Einkoppelrichtung (24) nach außen abdeckt.

3. Gerät nach Anspruch 1 oder 2, bei welchem die Schalldämpfungshülse (21) dazu ausgelegt ist, im Bereich eines dem Körper zugewandten offenen Endes (25) solchermaßen von dem Gehäuse (1) beabstandet zu sein, dass ein Schalldämpfungshülseninnenwandbereich (26) zusammen mit dem Gehäuse (1) ein Volumen begrenzt, in welches ein Koppelgel einfüllbar ist, vorzugsweise ein zu dem offenen Ende der Schalldämpfungshülse (21) hin offenes Volumen.

4. Gerät nach einem der vorstehenden Ansprüche, bei welchem ein dem Körper zugewandtes offenes Ende (25) der Schalldämpfungshülse (21) durch einen nach innen gerichteten umlaufenden Kragen (27) verjüngt ist.

5. Gerät nach einem der vorstehenden Ansprüche, bei welchem an der Schalldämpfungshülse (21) innenseitig ein Abstandshalter (22) vorgesehen ist, der dazu ausgelegt ist, die Schalldämpfungshülse (21) von dem Gehäuse (1) zu beabstanden und vorzugsweise als innenseitiger, besonders bevorzugt umlaufender Steg (22) ausgebildet ist.

6. Gerät nach Anspruch 6, bei welchem die Schalldämpfungshülse (21) dazu ausgelegt ist, ein geschlossenes, zwischen dem Gehäuse (1) und der Schalldämpfungshülse (21) angeordnetes Luftvolumen (23), vorzugsweise ein ringförmiges Luftvolumen (23), zu begrenzen.

7. Gerät nach einem der vorstehenden Ansprüche, bei welchem die Schalldämpfungshülse (21) einen Elastomerkörper (42a, b) umfasst und für eine solche Anlage an dem Gehäuse (1) ausgelegt ist, dass der Elastomerkörper (42a, b) zwischen dem Gehäuse (1) und der Schalldämpfungshülse (21) angeordnet ist.

8. Gerät nach einem der vorstehenden Ansprüche, bei welchem die Schalldämpfungshülse (21) aus einem Elastomermaterial vorgesehen ist.

9. Gerät nach einem der vorstehenden Ansprüche, bei welchem die Schalldämpfungshülse (21) bei Raumtemperatur einen Elastizitätsmodul von weniger als 10 GPa, vorzugsweise weniger als 5 GPa hat.

10. Gerät nach einem der vorstehenden Ansprüche, bei welchem die Schalldämpfungshülse (21) eine senkrecht zur Einkoppelrichtung (24) genommene Dicke von mindestens 1 mm hat.

11. Verwendung einer Schalldämpfungshülse (21) zum Aufsetzen auf ein Gerät, welches Gerät
einen beweglichen Schlagteil (13),
einen Prallkörper (9), wobei die Druckwellen durch Beschleunigen und Aufprallen des Schlagteils (13) auf den Prallkörper (9) erzeugbar und in den Körper einkoppelbar sind,
und ein Gehäuse (1), in dem das Schlagteil (13) und der Prallkörper (9) montiert sind, umfasst,
wobei die Schalldämpfungshülse (21) außenseitig an dem Gehäuse (1) anliegt und dabei in Einkoppelrichtung (24) eine Länge hat, die mindestens 20% der in derselben Richtung genommenen Länge eines sich innerhalb des Gehäuses (1) erstreckenden Teils des Prallkörpers (9) beträgt,
**dadurch gekennzeichnet, dass**
die Schalldämpfungshülse (21) als rohrförmiger Körper mit offenen Enden ausgebildet ist.

12. Verwendung nach Anspruch 11 für ein Gerät nach einem der Ansprüche 1 bis 10.

13. Verwendung nach Anspruch 11 oder 12, wobei die Schalldämpfungshülse (21) vor einer Behandlung eines weiteren Patienten gegen eine unbenutzte Schalldämpfungshülse (21) ausgetauscht wird.

14. Verwendung nach einem der Ansprüche 11 bis 13 eines Geräts nach Anspruch 3, wobei vor einer Behandlung in das von Schalldämpfungshülse (21) und Gehäuse (1) begrenzte Volumen ein Koppelgel eingefüllt wird.

## Claims

1. An apparatus for treating the human or animal body by mechanical shockwaves, having
a movable projectile (13),
an impact body (9), wherein said shockwaves can be generated and coupled into said body by accelerating said projectile (13) and by said projectile (13) hitting said impact body (9),
and a housing (1) in which said projectile (13) and said impact body (9) are mounted and
a sound absorbing sleeve (21),
wherein said sound absorbing sleeve (21) is adapted for contacting said housing (1) at the outside thereof and has a length measured in a shockwave coupling direction (24), which amounts to at least 20% of a length of that part of said impact body (9), which extends within said housing (1), said length of said part of said impact body (9) being taken in the same shockwave coupling direction (24),
**characterized in that**
said sound absorbing sleeve (21) is provided as a tube-like shaped body having open ends.

2. The apparatus according to claim 1, wherein said sound absorbing sleeve (21) is adapted for contacting said housing (1) in such a way that said sound absorbing sleeve (21) covers a region of said impact body (9), provided for being hitted by said projectile (13), namely covers it perpendicularly to said shockwave coupling direction (24) towards the outside.

3. The apparatus according to claim 1 or 2, wherein said sound absorbing sleeve (21) is, in a region at that open end (25) which faces said body, adapted for being distant to said housing (1) in such a way that an inner wall region (26) of said sound absorbing sleeve defines a volume together with said housing (1), into which a contact gel can be filled, wherein said volume is preferably open towards said open end of said sound absorbing sleeve (21).

4. The apparatus according to one of the preceding claims, wherein an open end (25) of said sound absorbing sleeve (21), which faces said body, is tapered by a collar (27) pointing inwards and extending circumferentially.

5. The apparatus according to one of the preceding claims, wherein a spacer (22) is provided at the sound absorbing sleeve (21) on the inside thereof, said spacer (22) being adapted for spacing said sound absorbing sleeve (21) from said housing (1), said spacer (22) being preferably provided as a bar (22) at the inside, which particularly preferred extends circumferentially.

6. The apparatus according to claim 5, wherein said sound absorbing sleeve (21) is adapted for defining an air volume (23) between said housing (1) and said sound absorbing sleeve (21), preferably a ring-shaped air volume (23).

7. The apparatus according to one of the preceding claims, wherein said sound absorbing sleeve (21) comprises an elastomer body (42a,b) and is adapted for contacting said housing (1) in such a way that said elastomer body (42a,b) is provided between said housing (1) and said sound absorbing sleeve (21).

8. The apparatus according to one of the preceding claims, wherein said sound absorbing sleeve (21) is made of an elastomer material.

9. The apparatus according to one of the preceding claims, wherein said sound absorbing sleeve (21) has a Young's modulus of less than 10 GPa at room temperature, preferably less than 5 GPa.

10. The apparatus according to one of the preceding claims, wherein said sound absorbing sleeve (21) has a thickness measured perpendicularly to said coupling direction (24) of at least 1 mm.

11. A use of a sound absorbing sleeve (21) for placing it up on an apparatus, said apparatus comprising
a movable projectile (13),
an impact body (9), wherein said shockwaves can be generated and coupled into the body by accelerating said projectile (13) and by said projectile (13) hitting said impact body (9),
and a housing (1) in which said projectile (13) and said impact body (9) are mounted,
wherein said sound absorbing sleeve (21) contacts said housing (1) at the outside thereof and has a length measured in a shockwave coupling direction (24), which amounts to at least 20% of a length of that part of said impact body (9), which extends within said housing (1), said length of said part of said impact body (9) being taken in the same shockwave coupling direction (24),
**characterized in that**
said sound absorbing sleeve (21) is provided as a tube-like shaped body having open ends.

12. The use according to claim 11 for an apparatus according to one of claims 1 to 10.

13. The use according to claim 11 or 12, wherein said sound absorbing sleeve (21) is replaced by an unused sound absorbing sleeve (21) prior to treating a further patient.

14. The use according to one of claims 11 to 13 of an apparatus according to claim 3, wherein a contact gel is added into said volume defined by said sound absorbing sleeve (21) and said housing (1) prior to a treatment.

## Revendications

1. Appareil pour le traitement du corps humain ou animal par des ondes de pression mécaniques, comportant
une partie percutante (13) mobile,
un corps d'impact (9), les ondes de pression pouvant être produites par accélération et percussion de la partie percutante (13) sur le corps d'impact (9) et être injectées dans le corps,
et un boîtier (1) dans lequel sont montés la partie percutante (13) et le corps d'impact (9), et
un manchon d'amortissement acoustique (21),
dans lequel le manchon d'amortissement acoustique (21) est dimensionné pour être mis en place sur l'extérieur du boîtier (1) et présente à cet effet une longueur, dans la direction d'injection (24), qui est au moins de 20 % celle de la longueur, considérée dans la même direction, d'une partie du corps d'impact (9) s'étendant à l'intérieur du boîtier (1),
**caractérisé en ce que**
le manchon d'amortissement acoustique (21) est conçu sous la forme d'un corps tubulaire à extrémités ouvertes.

2. Appareil selon la revendication 1, dans lequel le manchon d'amortissement acoustique (21) est dimensionné pour être mis en place sur le boîtier (1) de manière à recouvrir vers l'extérieur, perpendiculairement à la direction d'injection (24), une zone du corps d'impact (9) prévue pour la percussion de la partie percutante (13).

3. Appareil selon la revendication 1 ou 2, dans lequel le manchon d'amortissement acoustique (21) est dimensionné pour être espacé du boîtier (1), à proximité d'une extrémité ouverte (25) tournée vers le corps, de manière qu'une zone de paroi intérieure (26) du manchon d'amortissement acoustique délimite, conjointement avec le boîtier (1), un volume pouvant être rempli d'un gel de couplage et de préférence un volume ouvert vers l'extrémité ouverte du manchon d'amortissement acoustique (21).

4. Appareil selon l'une des revendications précédentes, dans lequel une extrémité ouverte (25), tournée vers le corps, du manchon d'amortissement acoustique (21) a une forme conique du fait d'une collerette (27) périphérique dirigée vers l'intérieur.

5. Appareil selon l'une des revendications précédentes, dans lequel il est prévu un espaceur (22), sur l'intérieur du manchon d'amortissement acoustique (21), qui est dimensionné pour espacer le manchon d'amortissement acoustique (21) par rapport au boîtier (1) et qui est conçu de préférence sous la forme d'une saillie (22) intérieure de préférence circulaire.

6. Appareil selon la revendication 5, dans lequel le manchon d'amortissement acoustique (21) est dimensionné pour délimiter un volume d'air (23) fermé situé entre le boîtier (1) et le manchon d'amortissement acoustique (21), de préférence un volume d'air (23) annulaire.

7. Appareil selon l'une des revendications précédentes, dans lequel le manchon d'amortissement acoustique (21) comprend un corps élastomère (42a, b) et est dimensionné pour une mise en place sur le boîtier (1) de manière que le corps élastomère (42a, b) soit disposé entre le boîtier (1) et le manchon d'amortissement acoustique (21).

8. Appareil selon l'une des revendications précédentes, dans lequel le manchon d'amortissement acoustique (21) est conçu en matériau élastomère.

9. Appareil selon l'une des revendications précédentes, dans lequel le manchon d'amortissement acoustique (21) présente, à température ambiante, un module d'élasticité inférieur à 10 GPa, de préférence inférieur à 5 GPa.

10. Appareil selon l'une des revendications précédentes, dans lequel le manchon d'amortissement acoustique (21) présente une épaisseur, considérée perpendiculairement à la direction d'injection (24), inférieure à 1 mm.

11. Utilisation d'un manchon d'amortissement acoustique (21) destiné à être mis en place sur un appareil, lequel appareil comprend
une partie percutante (13) mobile,
un corps d'impact (9), les ondes de pression pouvant être produites par accélération et percussion de la partie percutante (13) sur le corps d'impact (9) et être injectées dans le corps,
et un boîtier (1) dans lequel sont montés la partie percutante (13) et le corps d'impact (9), et
dans lequel le manchon d'amortissement acoustique (21) est mis en place sur l'extérieur du boîtier (1) et présente à cet effet une longueur, dans la direction d'injection (24), qui est au moins de 20 % celle de la longueur, considérée dans la même direction, d'une partie du corps d'impact (9) s'étendant à l'intérieur du boîtier (1),
**caractérisé en ce que**
le manchon d'amortissement acoustique (21) est conçu sous la forme d'un corps tubulaire à extrémités ouvertes.

12. Utilisation selon la revendication 11, pour un appareil selon l'une des revendications 1 à 10.

13. Utilisation selon la revendication 11 ou 12, dans laquelle, avant le traitement d'un autre patient, le manchon d'amortissement acoustique (21) est échangé contre un manchon d'amortissement acoustique (21) neuf.

14. Utilisation selon la revendication 11 à 13 d'un appareil selon la revendication 3, dans laquelle, avant un traitement, le volume délimité par le manchon d'amortissement acoustique (21) et le boîtier (1) est rempli d'un gel de couplage.
